(19) Europäisches Patentamt
European Patent Office
Office européen des brevets

(11) Publication number: 0 039 369
B1

(12) **EUROPEAN PATENT SPECIFICATION**

(45) Date of publication of patent specification: 15.06.83

(21) Application number: 80102385.4

(22) Date of filing: 02.05.80

(51) Int. Cl.³: C 07 J 5/00, A 61 K 9/72 //A61K31/57

# (54) Beclomethasone ester solvates, process for their preparation, and preparation of a formulation.

(43) Date of publication of application:
11.11.81 Bulletin 81/45

(45) Publication of the grant of the patent:
15.06.83 Bulletin 83/24

(84) Designated Contracting States:
AT BE CH DE FR GB IT LI NL SE

(56) References cited:
FR - A - 2 361 900
GB - A - 1 429 184

CHEMICAL ABSTRACTS, vol. 90, no. 24, 11th June 1979, column 2, page 367, no. 192558r Columbus, Ohio, U.S.A.

(73) Proprietor: SCHERING CORPORATION
2000 Galloping Hill Road
Kenilworth, New Jersey 07033 (US)

(72) Inventor: Finckenor, Lawrence Edward
15 Barbara Way
Wayne New Jersey 07470 (US)

(74) Representative: Antony, Fritz, Dr. et al,
P.O. Box 601 Winkelriedstrasse 35
CH-6002 Lucerne (CH)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 039 369 B1

# Beclomethasone ester solvates, process for their preparation, and preparation of a formulation

This invention relates to novel solvates of a beclomethasone ester, namely beclomethasone dipropionate, to processes for their preparation, and to their use in the preparation of aerosols.

Beclomethasone dipropionate is 9$\alpha$-chloro-16$\beta$-methyl-1,4-pregnadiene-11$\beta$,17$\alpha$,21-triol-3,20-dione 17$\alpha$,21-dipropionate and has the following structural formula:

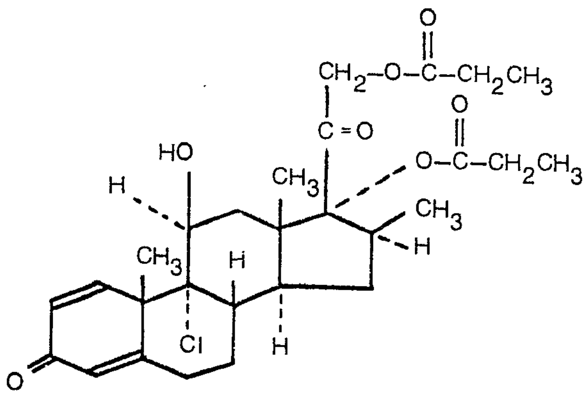

It is a useful drug for the treatment of chronic allergic asthma (*Brit. Med. J., 1,* 585—590 (1972)) and is typically administered in an aerosol unit containing a microcrystalline suspension of beclomethasone dipropionate in a propellant, usually trichlorofluoromethane. The drug must be micronized prior to use in an aerosol formulation in order to obtain particles of medicinally effective size. However, when unsolvated drug is introduced into the aerosol formulation, the micronised drug particles solvate and undergo crystal growth, which reduces the amount of drug of suitable particle size available in the spray and also causes the aerosol spray valves to clog. To overcome this problem of crystal growth, it has been found useful to prepare a solvate of the drug with the trichlorofluoromethane propellant prior to micronization of the drug (British Patent Specification No. 1,429,184). The drug solvate is then micronized and mixed with the remaining aerosol propellants. Although the beclomethasone dipropionate-trichlorofluoromethane solvate thus enables one to prepare a suitable aerosol form, it presents other manufacturing difficulties in that the solvate when stored as bulk is not stable with respect to trichlorofluoromethane. Trichlorofluoromethane is released when the solvate is stored at room temperature or above, and thus the solvate must be used rather promptly or be stored under refrigeration. Storage under refrigeration is both expensive and inconvenient, particularly when the drug solvate is to be shipped in bulk. Moreover, a substantial portion (up to one third) of the trichlorofluoromethane is lost from the trichlorofluoromethane solvate during the micronization. The loss of the trichlorofluoromethane from the drug solvate results in a loss of micronized drug in the aerosol formulation since (as mentioned above) any unsolvated drug will tend to undergo crystal growth and thus not be medicinally available in the aerosol spray. The lost trichlorofluoromethane is also a potential environmental hazard.

The present invention is based upon the surprising discovery that beclomethasone dipropionate forms solvates with alkanes which are substantially stable with respect to alkane when stored as bulk.

The present invention therefore provides solvates of beclomethasone dipropionate with alkanes having from 5 to 8 carbon atoms. For the purposes of this invention we define the term "solvate" as a crystalline material in which the steroid beclomethasone dipropionate and the alkane are associated. No particular method of association is implied, but it is possible that the alkane occupies "holes" in the crystal lattice of the steroid. The solvate normally contains from 4 to 8% by weight of alkane, the amount depending upon the particular method of preparation as well as upon the alkane itself. The alkane is preferably an *n*-alkane, i.e. *n*-pentane, *n*-heptane or *n*-octane, or especially *n*-hexane.

The accompanying drawing shows the infrared absorption spectrum of a mull of beclomethasone dipropionate-*n*-hexane solvate in mineral oil (sold under the Trade mark "Nujol"). In the drawing the vertical scale indicates the transmittance (shown by "T(%)"), and the horizontal scale indicates the frequency in $cm.^{-1}$ (shown by "$\nu(cm.^{-1})$") and the wavelength in microns (shown by "$\lambda(\mu)$"). This sample of solvate contained 4—5% of *n*-hexane by weight. Beclomethasone dipropionate-*n*-hexane solvate normally contains 4 to 6% by weight of *n*-hexane, in particular about 4.6% or 5.3% by weight of *n*-hexane.

The infrared spectra of beclomethasone dipropionate-*n*-pentane solvate and of beclomethasone dipropionate-*n*-heptane solvate are very similar to the spectrum shown in the accompanying drawing, in that all these spectra have absorption bands in almost identical positions. The relative intensities of some absorption bands may vary, depending upon the *n*-alkane in the solvate and how much of it is

present in the solvate. A particular solvate may also show a few absorption bands not characteristic of other solvates.

The beclomethasone dipropionate-alkane solvates are stable with respect to the alkane at moderate temperatures. In particular, beclomethasone dipropionate-*n*-hexane solvate is stable with respect to *n*-hexane at temperatures up to about 100°C.; *n*-hexane is first lost at about 105°C. (as shown by differential thermal analysis). The *n*-hexane solvate can however be broken at 80°C. under vacuum.

The solvates according to the invention can be prepared by intimately contacting beclomethasone dipropionate with an alkane having from 5 to 8 carbon atoms. Although it may be convenient under particular circumstances to contact micronised beclomethasone dipropionate with the alkane, it is generally preferable to allow the beclomethasone dipropionate to crystallise out of an organic solvent medium comprising the alkane. Although the organic solvent medium may be pure alkane (e.g. if the beclomethasone dipropionate is extracted out of a Soxhlet), it preferably comprises the alkane and an organic solvent that is completely miscible therewith, e.g. chloroform, tetrahydrofuran, dioxan, di-isopropyl ether, diethyl ether, ethyl acetate, cyclohexane, acetonitrile, isopropanol, or especially methylene chloride or acetone. The alkane is preferably an *n*-alkane, i.e. *n*-pentane, *n*-heptane or *n*-octane or especially *n*-hexane. The solvate is conveniently prepared by dissolving the beclomethasone dipropionate in a suitable organic solvent (such as one mentioned above) and then adding the alkane; preferably the original solvent is then at least partly removed by distillation, while further alkane is added to maintain total solvent volume. After cooling, the precipitated solvate is filtered off and dried.

The alkane solvates of this invention and in particular the *n*-hexane solvate of beclomethasone dipropionate are simpler to prepare than the known trichlorofluoromethane solvate, in that they can be prepared without using the large volumes of solvating medium that the trichlorofluoromethane solvate needs.

The novel beclomethasone dipropionate-alkane solvates are suitable for use in the preparation of beclomethasone dipropionate-trichlorofluoromethane solvate especially of an appropriate particle size (e.g. 1 to 10 $\mu$) for use as active ingredient of an aerosol formulation. The invention therefore provides a process for the preparation of beclomethasone dipropionate-trichlorofluoromethane solvate which comprises bringing a solvate of beclomethasone dipropionate with an alkane having 5 to 8 carbon atoms into contact with trichlorofluoromethane. Preferably the alkane solvate is micronised, so that the resulting trichlorofluoromethane solvate is also micronised. The method of preparation of beclomethasone dipropionate-trichlorofluoromethane solvate according to the present invention furthermore has the advantage of not requiring such large volumes of trichlorofluoromethane as are required by the known method. For example, when the beclomethasone dipropionate-*n*-hexane solvate is contacted with a relatively small volume of trichlorofluoromethane, e.g. 15 litres per kg, of solvated steroid, the *n*-hexane of solvation is exchanged for trichlorofluoromethane so that beclomethasone dipropionate-trichlorofluoromethane solvate is formed. (Preparation of the trichlorofluoromethane solvate by the method of British Patent Specification No. 1,429,184, Example 2, would require about 140 litres of trichlorofluoromethane per kg. of steroid.)

This process is preferably carried out on micronised beclomethasone dipropionate-*n*-hexane solvate so that micronised beclomethasone dipropionate-trichlorofluoromethane solvate is isolated. Alternatively and preferably, micronized beclomethasone dipropionate-alkane solvate (especially the *n*-hexane solvate) may be placed directly in the aerosol canister with the trichlorofluoromethane propellant to afford *in situ* an aerosol formulation in which the beclomethasone dipropionate does not exhibit significant crystal growth and exists in a particle size suitable for local absorption.

Since the beclomethasone dipropionate-trichlorofluoromethane solvate prepared by this method retains substantially the particle size of the micronized beclomethasone dipropionate-alkane solvate, it may be used directly without further micronization in an aerosol formulation, so that loss of trichlorofluoromethane and consequent crystal growth on resolvation can be avoided.

The amount of alkane (e.g. *n*-hexane) present in the solvate of this invention is considered biologically insignificant and thus the alkane solvate may be used directly in a formulation without toxic effects.

The aerosol propellants and valves suitable for use in this feature of the invention are standard and well known in the art. A particularly suitable inhaler is the inhaler presently marketed under the Trade Mark Vanceril.

The following examples illustrate but do not limit the present invention:

Example 1

*Beclomethasone Dipropionate-n-Hexane Solvate*

Dissolve 300 g. of beclomethasone dipropionate in 2 liters of methylene chloride at reflux. Treat with 15 g. activated charcoal for 15 minutes at reflux and filter while hot. Concentrate the filtered solution to a volume of 900 ml. and while maintaining reflux add slowly 900 ml. of *n*-hexane. Cool to 0—10°C. Filter off the resulting precipitate and wash it with *n*-hexane. Dry the precipitate in air below 50°C. to constant weight to afford beclomethasone dipropionate-*n*-hexane solvate having an $[\alpha]_D^{25} = +85.5° \pm 2°$ in dioxan and an $\varepsilon$1% (extinction coefficient) in 1% solution $= 275 \pm 10$ at

239 mμ. Analysis by gas chromatography shows an *n*-hexane content of 4.6%. The beclomethasone dipropionate content (determined by ultraviolet assay) is 94.5%.

Example 2

*Beclomethasone Dipropionate-n-Hexane Solvate*

Dissolve 100 g. of beclomethasone dipropionate in 1.5 liters of acetone at reflux. Treat with 5 g. of activated charcoal for 15 minutes and filter while hot. Concentrate the filtered solution to 0.5 liter. While maintaining reflux, slowly add 0.5 liter of *n*-hexane. Cool to 0—10°C. Filter off the resulting precipitate and wash it with cold *n*-hexane. Dry the precipitate in air at 50°C. to constant weight to yield beclomethasone dipropionate-*n*-hexane solvate. The beclomethasone dipropionate content (determined by ultraviolet assay) is 94.6%.

Example 3

*Beclomethasone Dipropionate-n-Hexane Solvate*

Under reflux, dissolve 20 g. of beclomethasone dipropionate in 100 ml. of methylene chloride. If necessary, treat with activated charcoal and filter. Slowly add 400 ml. of *n*-hexane while distilling at a rate that maintains a batch volume of 100 ml. Reflux the batch at a volume of 100 ml. (b.p. 68°C.) for half an hour. Cool slowly to 0—5°C.; filter, wash the precipitate with *n*-hexane, and dry it at 60°C. to constant weight; yield 21 g. *n*-Hexane by gas chromatography assay (two samples): 5.29%; 5.07%.

Example 4

*Beclomethasone Dipropionate-n-Hexane Solvate*

Under reflux, dissolve 50 g. of beclomethasone dipropionate in 250 ml. of acetone. If necessary, treat with activated charcoal and filter. Distil at a slow rate adding *n*-hexane just fast enough to maintain the original batch volume. Continue the distillation until 1 liter of *n*-hexane has been added and the batch has a volume of 250 ml. and a boiling point of 68°C. Reflux the batch for half an hour and cool it slowly to 0—5°C. Filter off the precipitate, wash it with *n*-hexane, and dry it in the air at 60°C. to constant weight; yield 52.8 g. *n*-Hexane by gas chromatography assay (three samples): 5.34%; 5.33%; 5.78%.

Example 5

*Beclomethasone Dipropionate-n-Pentane Solvate*

Under reflux, dissolve 2 g. of beclomethasone dipropionate in 15 ml. of methylene chloride. Add 10 ml. of *n*-pentane and concentrate to 15 ml. Continue the addition/concentration sequence until 150 ml. of pentane has been added and the batch has a volume of 15 ml. and a boiling point of 36°C. Cool slowly to 0—5°C. Filter off the precipitate, wash it with *n*-pentane, and dry it at 60°C. in air to constant weight; yield 1.99 g., $[\alpha]_D^{26}$ (dioxan) +88.9°, ε1% in MeOH = 285 at 238 mμ. *n*-Pentane by gas chromatography assay: 4.39%.

Example 6

*Beclomethasone Dipropionate-n-Heptane Solvate*

Under reflux dissolve 2 g. of beclomethasone dipropionate in 25 ml. of methylene chloride. Add 7 ml. of *n*-heptane and bring to reflux under partial vacuum. Slowly add 140 ml. of *n*-heptane while distilling under reduced pressure so as to maintain a volume of 25 ml. Stir for half an hour at room temperature. Cool to 0—5°C., filter off the precipitate, wash it with *n*-heptane and dry it in air at 60°C. to constant weight; yield 2.1 g., $[\alpha]_D^{26}$ (dioxan) = +85.5°, ε1% in MeOH = 277 at 238 mμ. *n*-Heptane by gas chromatography assay: 7.32%.

FORMULATION EXAMPLE

*Beclomethasone Dipropionate Inhaler*

| *Formula* | *mg/container (200 doses)* |
|---|---|
| Beclomethasone Dipropionate* (micronized) | 10.0 |
| Oleic Acid | 1.0 |
| Trichlorofluoromethane | 4,739.0 |
| Dichlorodifluoromethane | 12,250.0 |
| to make | 17,000.0 |

*Charged as beclomethasone dipropionate-*n*-hexane solvate equivalent to 10 mg. of beclomethasone dipropionate.

*Procedure*

Add oleic acid to previously cooled trichlorofluoromethane and mix with a high-sheer mixer. While mixing, add the required amount of beclomethasone dipropionate-*n*-hexane solvate and continue mixing until homogeneous. If necessary, adjust the suspension to the required weight with trichlorofluoromethane. Meter the required amount of suspension into each can. Crimp the valves onto the cans. Pressure-fill through the valves the required amount of dichlorodifluoromethane.

**Claims for the Contracting States: BE CH LI DE FR GB IT NL SE**

1. Solvates of belcomethasone dipropionate with alkanes having from 5 to 8 carbon atoms, especially with *n*-alkanes such as *n*-pentane and *n*-heptane and in particular with *n*-hexane, and preferably containing from 4 to 8% by weight of alkane.

2. Beclomethasone dipropionate-*n*-hexane solvate containing from 4 to 6%, especially about 4.6% or about 5.3%, by weight of *n*-hexane, and in particular having an infrared spectrum on a suspension thereof in mineral oil substantially as shown in the accompanying drawing.

3. A process for the preparation of a solvate as claimed in claim 1 which comprises intimately contacting beclomethasone dipropionate with an alkane having from 5 to 8 carbon atoms, especially by allowing beclomethasone dipropionate to crystallise out of an organic solvent medium comprising an alkane having from 5 to 8 carbon atoms.

4. A process as claimed in claim 3 wherein the organic solvent medium comprises the alkane and an organic solvent that is completely miscible with the alkane, such as chloroform, tetrahydrofuran, dioxan, di-isopropyl ether, diethyl ether, ethyl acetate, cyclohexane, acetonitrile or isopropanol, or especially methylene chloride or acetone.

5. A process as claimed in claim 3 or claim 4 wherein the alkane is *n*-pentane or *n*-heptane or especially *n*-hexane.

6. A process for the preparation of beclomethasone dipropionate-trichlorofluoromethane solvate, which comprises bringing a solvate as claimed in claim 1 or beclomethasone dipropionate with an alkane having 5 to 8 carbon atoms into contact with trichlorofluoromethane.

7. A process for the preparation of micronised beclomethasone dipropionatetrichlorofluoromethane solvate which comprises bringing a micronised solvate of beclomethasone dipropionate with an alkane having 5 to 8 carbon atoms, especially *n*-hexane, into contact with trichlorofluoromethane.

8. A process as claimed in claim 7 wherein the beclomethasone dipropionate-trichlorofluoromethane solvate is formed *in situ* in an aerosol formulation.

9. A method of preparing an aerosol formulation of beclomethasone dipropionate which comprises the step of placing a micronised solvate claimed in claim 1, especially beclomethasone dipropionate-*n*-hexane solvate, in an aerosol propellant, especially an aerosol propellant comprising trichlorofluoromethane.

**Claims for the Contracting State: AT**

1. A process for the preparation of solvates of beclomethasone dipropionate with alkanes having from 5 to 8 carbon atoms, which comprises intimately contacting beclomethasone dipropionate with an alkane having from 5 to 8 carbon atoms.

2. A process as claimed in claim 1, wherein beclomethasone dipropionate is allowed to crystallise out of an organic solvent medium comprising an alkane having from 5 to 8 carbon atoms.

3. A process as claimed in claim 2 wherein the organic solvent medium comprises the alkane and an organic solvent that is completely miscible with the alkane, such as chloroform, tetrahydrofuran, dioxan, di-isopropyl ether, diethyl ether, ethyl acetate, cyclohexane, acetonitrile or isopropanol, or especially methylene chloride or acetone.

4. A process as claimed in any of claims 1 to 3 wherein the alkane is an *n*-alkane, in particular *n*-pentane or *n*-heptane or especially *n*-hexane.

5. A process as claimed in any of claims 1 to 4 wherein the resulting solvate is isolated as a solvate containing from 4 to 8% by weight of alkane.

6. A process as claimed in any of claims 1 to 4 wherein the resulting solvate is isolated as a solvate containing from 4 to 6% by weight of *n*-hexane.

7. A process for the preparation of beclomethasone dipropionate-trichlorofluoromethane solvate, which comprises bringing a solvate as produced in any of claims 1 to 6 of beclomethasone dipropionate with an alkane having 5 to 8 carbon atoms into contact with trichlorofluoromethane.

8. A process for the preparation of micronised beclomethasone dipropionate-trichlorofluoromethane solvate with comprises bringing a micronised solvate of beclomethasone dipropionate with an alkane having 5 to 8 carbon atoms, especially *n*-hexane, into contact with trichlorofluoromethane.

9. A process as claimed in claim 8 wherein the beclomethasone dipropionate-trichlorofluoromethane solvate is formed *in situ* in an aerosol formulation.

10. A method of preparing an aerosol formulation of beclomethasone dipropionate which

comprises the step of placing a micronised solvate as produced in any of claims 1 to 6, especially beclomethasone dipropionate-*n*-hexane solvate, in an aerosol propellant, especially an aerosol propellant comprising trichlorofluoromethane.

**Patentansprüche für die Vertragsstaaten : BE CH LI DE FR GB IT NL SE**

1. Solvate von Beclomethason-dipropionat mit Alkanen, die 5 bis 8 Kohlenstoff-Atome besitzen, besonders mit n-Alkanen wie n-Pentan und n-Heptan und insbesondere mit n-Hexan, enthaltend vorzugsweise 4 bis 8 Gew.-% des Alkans.

2. Beclomethason-dipropionat-n-Hexan-Solvat, enthaltend 4 bis 6 Gew.-%, besonders etwa 4,6 oder etwa 5,3 Gew.-%, und insbesondere ein solches mit einem Infrarot-Spektrum einer Suspension desselben in Mineralöl, das im wesentlichen dem in der beigefügten Zeichnung dargestellten Infrarot-Spektrum entspricht.

3. Verfahren zur Herstellung eines Solvats nach Anspruch 1, dadurch gekennzeichnet, daß Beclomethason-dipropionat mit einem Alkan, das 5 bis 8 Kohlenstoff-Atome besitzt, in innige Berührung gebracht wird, insbesondere in der Weise, daß man Beclomethason-dipropionat aus einem organischen Lösungsmittel-Medium auskristallisieren läßt, das ein Alkan mit 5 bis 8 Kohlenstoff-Atomen enthält.

4. Verfahren nach Anspruch 3, dadurch gekennzeichnet, daß das organische Lösungsmittel-Medium aus dem Alkan und einem organischen Lösungsmittel besteht, das mit dem Alkan vollständig mischbar ist, wie Chloroform, Tetrahydrofuran, Dioxan, Diisopropyläther, Diäthyläther, Aethylacetat, Cyclohexan, Acetonitril oder Isopropanol oder insbesondere Methylenchlorid oder Aceton.

5. Verfahren nach Anspruch 3 oder 4, dadurch gekennzeichnet, daß das Alkan n-Pentan oder n-Heptan oder insbesondere n-Hexan ist.

6. Verfahren zur Herstellung eines Beclomethason-dipropionat-Trichlorfluormethan-Solvats, dadurch gekennzeichnet, dass ein Solvat nach Anspruch 1 von Beclomethason-dipropionat mit einem Alkan, das 5 bis 8 Kohlenstoff-Atome besitzt, mit Trichlorfluormethan in Berührung gebracht wird.

7. Verfahren zur Herstellung eines mikronisierten Beclomethason-dipropionat-Trichlorfluormethan-Solvats, dadurch gekennzeichnet, daß ein mikronisiertes Solvat von Beclomethason-dipropionat mit einem Alkan, das 5 bis 8 Kohlenstoff-Atome besitzt, insbesondere mit n-Hexan, mit Trichlorfluormethan in Berührung gebracht wird.

8. Verfahren nach Anspruch 7, dadurch gekennzeichnet, daß das Beclomethason-dipropionat-Trichlorfluormethan-Solvat in situ in einer Aerosol-Formulierung gebildet wird.

9. Vefahren zur Herstellung einer Aerosol-Formulierung von Beclomethason-dipropionat, gekennzeichnet durch den Arbeitsgang, in dem ein mikronisiertes Solvat nach Anspruch 1, insbesondere Beclomethason-dipropionat-n-Hexan-Solvat, in ein Aerosol-Treibmittel, insbesondere ein Trichlorfluormethan enthaltendes Aerosol-Treibmittel, gegeben wird.

**Patentansprüche für der Vertragsstaat: AT**

1. Verfahren zur Herstellung von Solvaten von Beclomethason-dipropionat mit Alkanen, die 5 bis 8 Kohlenstoff-Atome besitzen, dadurch gekennzeichnet, daß Beclomethason-dipropionat mit einem Alkan, das 5 bis 8 Kohlenstoff-Atome besitzt, in innige Berührung gebracht wird.

2. Verfahren nach Anspruch 1, dadurch gekennzeichnet, daß man Belcomethason-dipropionat aus einem organischem Lösungsmittel-Medium auskristallisieren läßt, das ein Alkan mit 5 bis 8 Kohlenstoff-Atomen enthält.

3. Verfahren nach Anspruch 2, dadurch gekennzeichnet, daß das organische Lösungsmittel-Medium aus dem Alkan und einem Lösungsmittel besteht, das mit dem Alkan vollständig mischbar ist, wie Chloroform, Tetrahydrofuran, Dioxan, Diisopropyläther, Diäthyläther, Aethylacetat, Cyclohexan, Acetonitril oder Isopropanol oder insbesondere Methylenchlorid oder Aceton.

4. Verfahren nach Anspruch 1 bis 3, dadurch gekennzeichnet, daß das Alkan ein n-Alkan, insbesondere n-Pentan oder n-Heptan oder vor allem n-Hexan ist.

5. Verfahren nach Anspruch 1 bis 4, dadurch gekennzeichnet, daß das resultierende Solvat als ein 4 bis 8 Gew.-% Alkan enthaltendes Solvat isoliert wird.

6. Verfahren nach Anspruch 1 bis 4, dadurch gekennzeichnet, daß das resultierende Solvat als ein 4 bis 6 Gew.-% n-Hexan enthaltendes Solvat isoliert wird.

7. Verfahren zur Herstellung eines Beclomethason-dipropionat-Trichlorfluormethan-Solvats, dadurch gekennzeichnet, daß ein nach Anspruch 1 bis 6 hergestelltes Solvat von Beclomethason-dipropionat mit einem Alkan, das 5 bis 8 Kohlenstoff-Atome besitzt, mit Trichlorfluormethan in Berührung gebracht wird.

8. Verfahren zur Herstellung eines mikronisierten Beclomethason-dipropionat-Trichlorfluormethan-Solvats, dadurch gekennzeichnet, daß ein mikronisiertes Solvat von Beclomethason-dipropionat mit einem Alkan, das 5 bis 8 Kohlenstoff-Atome besitzt, insbesondere mit n-Hexan, mit Trichlorfluormethan in Berührung gebracht wird.

9. Verfahren nach Anspruch 8, dadurch gekennzeichnet, daß das Beclomethason-dipropionat-Trichlorfluormethan-Solvat in situ in einer Aerosol-Formulierung gebildet wird.

10. Verfahren zur Herstellung einer Aerosol-Formulierung von Beclomethason-dipropionat, gekennzeichnet durch den Arbeitsgang, in dem ein nach Anspruch 1 bis 6 hergestelltes mikronisiertes Solvat, insbesondere Beclomethason-dipropionat-n-Hexan-Solvat, in ein Aerosol-Treibmittel, insbesondere ein Trichlorfluormethan enthaltendes Aerosol-Treibmittel, gegeben wird.

**Revendications pour les Etats contractants: BE CH LI DE FR GB IT NL SE**

1. Solvats de dipropionate de béclométhasone avec des alcanes, ayant de 5 à 8 atomes de carbone, spécialement avec des n-alcanes comme le n-pentane et le n-heptane et en particulier avec le n-hexane, et contenant de préférence de 4 à 8% en poids d'alcane.

2. Solvat de dipropionate de béclométhasone-n-hexane contenant de 4 à 6%, en particulier environ 4,6% ou environ 5,3% en poids de n-hexane, et ayant en particulier un spectre infrarouge sur sa suspension dans l'huile minérale qui est sensiblement tel que représenté sur le dessin joint.

3. Procédé de préparation d'un solvat selon la revendication 1 qui consiste à mettre en contact intime du dipropionate de béclométhasone avec un alcane ayant de 5 à 8 atomes de carbone, en particulier en laissant le dipropionate de béclométhasone cristalliser hors d'un milieu organique solvant comprenant un alcane ayant de 5 à 8 atomes de carbone.

4. Procédé selon la revendication 3 où le solvant organique comme milieu comprend l'alcane et un solvant organique qui est totalement miscible avec l'alcane, comme le chloroforme, le tétrahydrofurane, le dioxane, le diisopropyl éther, le diéthyl éther, l'acétate d'éthyle, le cyclohexane, l'acétonitrile ou l'isopropanol, et en particulier le chlorure de méthylène ou l'acétone.

5. Procédé selon la revendication 3 ou la revendication 4, où l'alcane est du n-pentane ou du n-heptane ou en particulier du n-hexane.

6. Procédé de préparation d'un solvat de dipropionate de béclométhasone-trichlorofluorométhane qui consiste à amener un solvat selon la revendication 1 de dipropionate de béclométhasone avec un alcane ayant de 5 à 8 atomes de carbone en contact avec du trichlorofluorométhane.

7. Procédé de préparation d'un solvat micronisé de dipropionate de béclométhasone-trichlorofluorométhane qui consiste à amener un solvat micronisé de dipropionate de béclométhasone avec un alcane ayant 5 à 8 atomes de carbone, en particulier du n-hexane, en contact avec du trichlorofluorométhane.

8. Procédé selon la revendication 7 où le solvat de dipropionate de béclométhasone-trichlorofluorométhane est formé *in situ* en une formule d'aérosol.

9. Procédé de préparation d'une formule d'aérosol de dipropionate de béclométhasone qui comprend l'étape de placer un solvat micronisé selon la revendication 1, en particulier un solvat de dipropionate de béclométhasone-n-hexane, dans un propulseur d'aérosol, en particulier un propulseur d'aérosol comprenant du trichlorofluorométhane.

**Revendications pour l'Etat contractant: AT**

1. Procédé de préparation de solvats de dipropionate de béclométhasone avec des alcanes ayant de 5 à 8 atomes de carbone, qui consiste à mettre en contact intime du dipropionate de béclométhasone avec un alcane ayant de 5 à 8 atomes de carbone.

2. Procédé selon la revendication 1, où on laisse le dipropionate de béclométhasone cristalliser hors d'un solvant organique comme milieu qui comprend un alcane ayant de 5 à 8 atomes de carbone.

3. Procédé selon la revendication 2, où le solvant organique comme milieu comprend l'alcane et un solvant organique qui est totalement miscible avec l'alcane, comme du chloroforme, du tétrahydrofurane, du dioxane, du diisopropyl éther, du diéthyl éther, de l'acétate d'éthyle, du cyclohexane, de l'acétonitrile ou de l'isopropanol, ou en particulier du chlorure de méthylène ou de l'acétone.

4. Procédé selon l'une quelconque des revendications 1 à 3, où l'alcane est un n-alcane, en particulier n-pentane ou n-heptane ou spécialement n-hexane.

5. Procédé selon l'une quelconque des revendications 1 à 4, où le solvat résultant est isolé en tant que solvat contenant de 4 à 8% en poids d'alcane.

6. Procédé selon l'une quelconque des revendications 1 à 4, où le solvat résultant est isolé sous forme d'un solvat contenant de 4 à 6% en poids de n-hexane.

7. Procédé de préparation d'un solvat de dipropionate de béclométhasone-trichlorofluorométhane qui consiste à amener un solvat tel que produit selon l'une quelconque des revendications 1 à 6 de dipropionate de béclométhasone avec un alcane ayant 5 à 8 atomes de carbone en contact avec du trichlorofluorométhane.

8. Procédé de préparation d'un solvat micronisé de dipropionate de béclométhasone-trichlorofluorométhane qui consiste à amener un solvat micronisé de dipropionate de béclométhasone avec un alcane ayant 5 à 8 atomes de carbone, en particulier du n-hexane, en contact avec du trichlorofluorométhane.

9. Procédé selon la revendication 8, où le solvat de dipropionate de béclométhasone-trichlorofluorométhane est formé *in situ* en une formule en aérosol.

10. Procédé de préparation d'une formule d'aérosol de dipropionate de béclométhasone qui comprend l'étape de placer un solvat micronisé tel que produit selon l'une quelconque des revendications 1 à 6, en particulier un solvat de dipropionate de béclométhasone-n-hexane, dans un propulseur d'aérosol, en particulier un propulseur d'aérosol comprenant du trichlorofluorométhane.

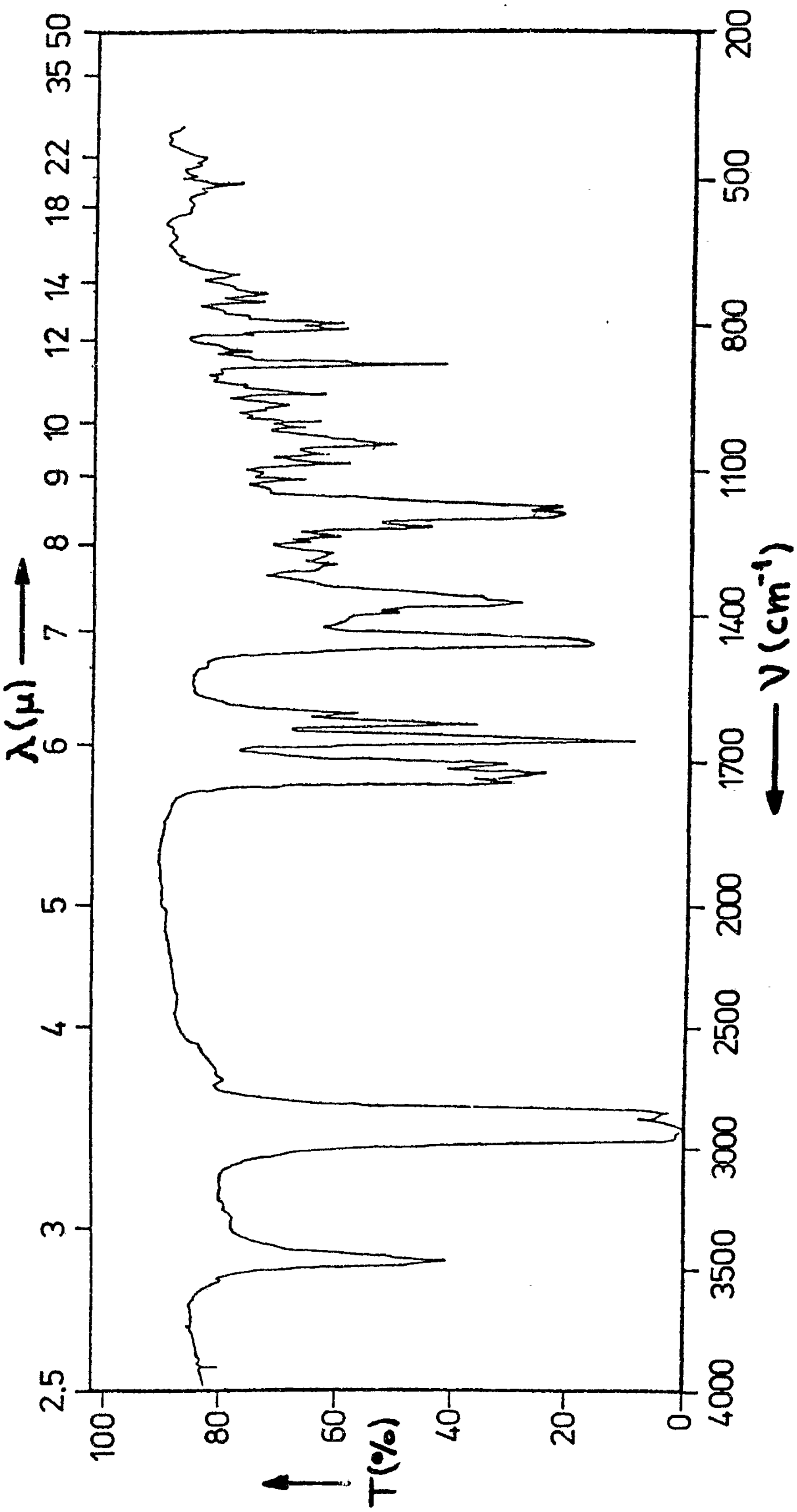
λ(μ)
2.5
3
4
5
6
7
8
9
10
12
14
18
22
35 50
ν (cm⁻¹)
4000
3500
3000
2500
2000
1700
1400
1100
800
500
200
T(%)
100
80
60
40
20
0